# EUROPÄISCHE PATENTSCHRIFT

(11) **EP 1 187 598 B1**
(45) Veröffentlichungstag und Bekanntmachung des Hinweises auf die Patenterteilung: **21.10.2009**
(21) Anmeldenummer: 00949173.9
(22) Anmeldetag: 08.06.2000
(51) Int. Cl.: A61Q 17/04, A61K 8/49

(54) **MISCHUNGEN AUS MIKROPIGMENTEN**
MICROPIGMENT MIXTURE
MELANGES DE MICROPIGMENTS

(30) Priorität: 18.06.1999 EP 99810543
(43) Veröffentlichungstag der Anmeldung: 20.03.2002
(73) Patentinhaber: BASF SE, 67056 Ludwigshafen (DE)
(72) Erfinder: LUTHER, Helmut, D-79639 Grenzach-Wyhlen (DE)
(86) Internationale Anmeldenummer: PCT/EP2000/005314
(87) Internationale Veröffentlichungsnummer: WO 2000/078277

(56) Entgegenhaltungen:
- EP-A- 0 582 189
- EP-A- 0 654 469
- EP-A- 0 818 450
- EP-A- 0 821 939
- WO-A-97/00851
- US-A- 5 338 539
- US-A- 5 445 815
- US-A- 5 518 713
- US-A- 5 601 811
- DR U. SCHÖFFLING: "Arzneiformenlehre" StartDateMarker 1998, EndDateMarker
- W. RAAB UND AL.: "Pflegekosmetic" StartDateMarker 1999, EndDateMarker

## Beschreibung

Die vorliegende Erfindung betrifft die Verwendung von Mischungen aus mikronisierten organischen UV-Filtern zur Herstellung eines Medikaments zum Schützen der menschlichen und tierischen Haut und Haare vor der schädigenden Einwirkung von UV-Strahlung und ihre Verwendung in kosmetischen und in pharmazeutischen Formulierungen.

Es ist bekannt, dass bestimmte organische UV-Filter, wie z.B. schwerlösliche Benzatriazol- oder Triazinverbindungen ausgeprägte UV-Filtereigenschaften aufweisen, wenn sie als Einzelverbindungen in mikronisierter Form vorliegen. Auf Grund ihrer spezifischen, substanztypischen Eigenschaften absorbieren, reflektieren bzw. streuen sie allerdings immer nur einen bestimmten Teil des schädlichen UV-Bereiches.

Es besteht ein starkes Interesse an Lichtschutzfiltern, die ein breites UV-Spektrum abdecken und damit einen besseren UV-Schutz bieten.

Die Aufgabe der vorliegenden Erfindung besteht also darin, mikronisierte organische UV-Filter zu finden, die einen breiteren Teil des UV-Bereichs abdecken und mit denen damit ein besserer UV-Schutz erzielt werden kann.

Überraschenderweise wurde nun gefunden, dass Mischungen aus mindestens zwei mikronisierten ausgewählt aus Triazin- und Benzotriazol-Derivaten wie in Anspruch 1 offenbart UV-Filtern diese Aufgabe erfüllen können.

Gegenstand der vorliegenden Erfindung ist daher die Verwendung von Mischungen aus
- Mischungen aus Octyltriazon und Methylene_Bis benzotriazolyltetramethylbutylphenol;
- Mischungen aus 2-[(2,4-methoxy)-phenyl]-4,6-bis-[(2-hydroxy-4-methoxy)-phenyl]-(1,3,5)-triazin und Methylen-bis-benzotriazolyltetramethylbutylphenol;
- Mischungen aus Methylen-bis-benzotriazolyltetramethylbutylphenol und Dioctybutamidotriazon;
- Mischungen aus 2,2'-Methylen-bis-[6-(2H-benztriazol-2-yl)-4-methyl-phenol, Octyltriazon und der Verbindung der Formel (36) 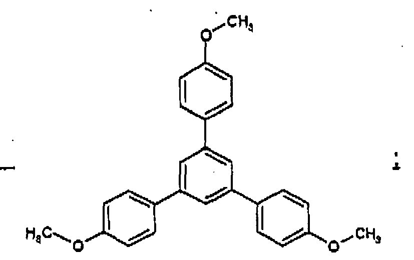
- Mischungen aus 2,2'-Methylen-bis-[6-(2H-benztriazol-2-yl)-4-methyl-phenol, Octyltriazon und der Verbindung der Formel (37) 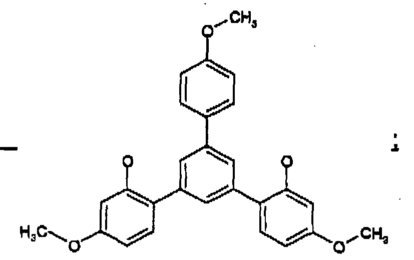
- Mischungen aus Methylen-bis-benzotriazolyltetramethylbutylphenol und der Verbindung der Formel (39) 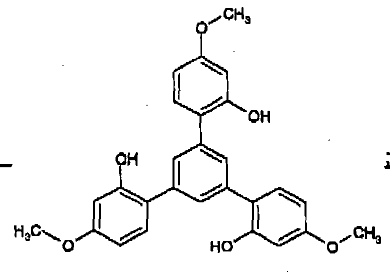 und
- Mischungen aus Methylen-bis-benzotriazolyltetramethylbutylphenol, Dioctylbutamidotriazon und der Verbindung der Formel (37) 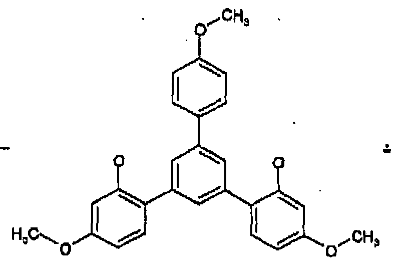
zur Herstellung eines Medikaments zum Schützen der menschlichen und tierischen Haut und der Haare vor der schädigenden Einwirkung von UV-Strahlung, wobei die mikronisierten organischen UV-Filter eine Partikelgrösse von 0,02 bis 2 µm aufweisen.
mikronisierten organischen UV-Filtern zum Schutz der menschlichen und tierischen Haut und Haare vor der schädlichen Einwirkung von UV-Strahlung.

Vorzugsweise werden Mischungen aus Methylene Bis-benzotriazolyltetramethylbutylphenol und Octyltriazon;
- Mischungen aus 2-[(2,4-methoxy)-phenyl]-4,6-bis-[(2-hydroxy-4-methoxy)-phenyl]-(1,3,5)-triazin und Methylen-bis-benzotriazolyltetramethylbutylphenol;
- Mischungen aus Methylen-bis-benzotriazolyltetramethylbutylphenol und Dioctybutamidotriazon; und
- Mischungen aus Methylen-bis-benzotriazolyltetramethylbutylphenol und der Verbindung der Formel (39) 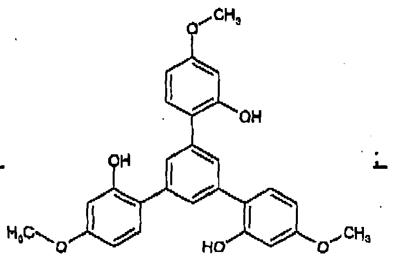
verwendet, und ganz besonders Mischungen_ aus Methylene Bis-benzotriazolyltetramethylbutylphenol und Octyltriazon.

Die Herstellung der erfindungsgemäss verwendbaren Mischungen mikronisierter organischer UV-Filter kann auf verschiedene Weise erfolgen.

Einerseits können mindestens zwei der oben erwähnten organischen UV-Filter als Einzelsubstanzen beim Herstellungsprozess der Mikropartikel (Mikronisierung) gemischt werden.

Eine weitere Herstellungsmöglichkeit besteht darin, dass die bereits mikronisierten Einzelsubstanzen der UV-Filter innig miteinander gemischt werden.

Eine dritte Möglichkeit der Herstellung besteht darin, dass man mindestens zwei der oben erwähnten UV-Filter zusammenschmilzt. Nach Abkühlen der Schmelze entsteht ein homogener Composite, der auf übliche Art und Weise mikronisiert wird.

Die homogenen Composites aus mindestens zwei organischen UV-Filtern bilden einen weiteren Erfindungsgegenstand.

Composites sind durch Einschmelzen von einem oder mehreren anorganischen Mikropigmenten in einen oder mehrere organische UV-Filter erhältlich.

Beispielhafte Mikropigmente sind z.B. TiO₂, ZnO, Eisenoxide oder andere anorganische Oxide, Glimmer (Mica) oder andere geeignete anorganische Mineralien, ferner auch Ti-, Erdalkali- oder Zinksalze von organischen Säuren.

Dadurch können gleichzeitig die unerwünschten photokatalytischen Eigenschaften einiger dieser anorganischen Mikropigmente (TiO₂, ZnO) unterdrückt werden und ihre positiven Eigenschaften zusätzlich voll genutzt werden.

Vorteilhafterweise werden die oben genannten anorganischen UV-Filter in Methylen-bis-benzotriazolyltetramethylbutylphenol eingeschmolzen. Der so entstandene Composite wird anschliessend auf übliche Weise mikronisiert.

Composites sind durch Schmelzen von mindestens zwei elektrisch neutralen organischer UV-Filten mit kationisch oder anionisch geladenen Verbindungen erhältlich.

Dazu werden kationisch oder anionisch geladene Verbindungen mit den entsprechenden organischen, elektrisch neutralen UV-Filtern geschmolzen und anschliessend abgekühlt. Durch dieses Verfahren lassen sich im anschliessenden Mikronisierungsschritt organische UV-Filterpigmente mit einer Permanentausrüstung aus einer positivenbzw. negativen Ladung herstellen. Eine solche Ausrüstung verhindert wirkungsvoll die Aggregation der mikronisierten Teilchen in den Sonnenschutzpräparaten, die bei einem Teilchendurchmesser von < 1 µm auftreten kann. Eine sonst übliche "Coatung" dieser Teilchen mit abstossender Wirkung wird dann zum Teil überflüssig.

Als kationisch oder anionisch geladene Verbindungen können UV-Filter oder auch andere Verbindungen verwendet werden, die eine oder mehrere kationische oder anionische Gruppierungen aufweisen, wie z.B.
- N,N,N-Trimethyl-4-(2-oxoborn-3-ylidenmethyl)-anilinmethylsulfat;
- Campherbenzalkoniummethosulfat;
- Fettamine;
- Betaine, wie z.B. Cocoamidopropylbetain;
- Quats, wie z.B. Ricinolamidopropyltrimodiummethosulphat, Quarternium 18 , oder Cetyltrimethylammoniumbromid;
- Behensäure und andere organische Säuren, wie z.B. Isostearinsäure, Zitronensäuremonoglycerid oder Natriummethylcocoyltaurat;
- Phospholipide, wie z.B. Phosphatidylcholin, Phosphatidylserin oder Alkylaminoxid;
- Ceramide und Pseudoceramide und Phytosterole.

Die letztgenannten Verbindungen verleihen den mikronisierten UV-Filtern eine oleophobe Ausrüstung.

Der Anteil der kationischen oder anionischen Verbindungen im Composite liegt zwischen 0,001 und 5, vorzugsweise 0,01 bis 3 Gew.-%, bezogen auf das Gewicht des oder der UV-Filter.

Dazu werden der/die schwerlösliche(n) oder unlösliche(n) organische(n) UV-Filter mit Antioxidantien zusammengeschmolzen, abgekühlt und anschliessend auf übliche Weise mikronisiert.

Als erfindungsgemäss verwendbare Antioxidantien kommen alle organischen Substanzen mit Scavenger-Eigenschaften in Betracht, die sich zusammen mit organischen UV-Filtern schmelzen lassen. Man erhält neuartige Mikropigmente, die gleichzeitig UV-Schutz und antioxidative Wirkung auf ihrer Oberfläche bieten. Diese Eigenschaft ist beim kosmetischen Sonnenschutz erwünscht, da unter UV- und Lichteinfluss sowohl in Formulierungen als auch auf der Haut schädliche Radikale gebildet werden können. Diese können z.B. zur sogenannten Mallorca-Akne oder zu vorzeitiger Hautalterung führen. Durch Ausrüstung der mikronisierten UV-Filter mit Antioxidantien wird neben dem Schutz vor UV-Schäden gleichzeitig ein Schutz gegen den photochemischen Abbau von Bestandteilen in der Sonnenschutzformulierung erreicht.

Der Anteil der Antioxidantien im Composite liegt dabei gewöhnlich zwischen 0,001 und 30, vorzugsweise 0,01 bis 3 Gew.-%, bezogen auf das Gewicht des/der UV-Filter.

Besonders vorteilhaft ist ein Anteil von Antioxidantien in Mikropigmenten, wenn diese neben organischen UV-Filtern die oben erwähnten photokatalytisch aktiven anorganischen Mikropigmente, wie z.B. Titandioxid, Zinkoxid (auch gecoatet) oder andere geeignete anorganischen Oxide, wie z.B. Eisenoxid enthalten.

Als beispielhafte Antioxidantien seien die folgenden Verbindungen aufgeführt:
- Tocopherole, wie z.B. α-Tocopherol (CAS 59-02-9), Tocopherylacetat, Vitamin E Succinat,
- Ellagsäure 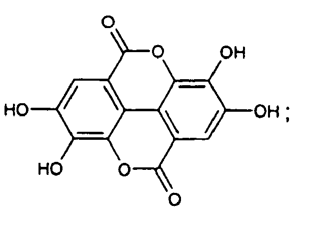
- Propylgallat (CAS 121-79-9) 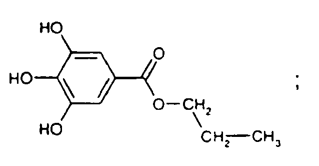
- N butyliertes Hydroxytoluol (BHT; CAS 128-37-0);
- butyliertes Hydroxyanisol (BHA);
- 2,4,6-Tris(3,5-di-t-butyl-4-hydroxybenzyl)mesitylen (CAS 1709-70-2) 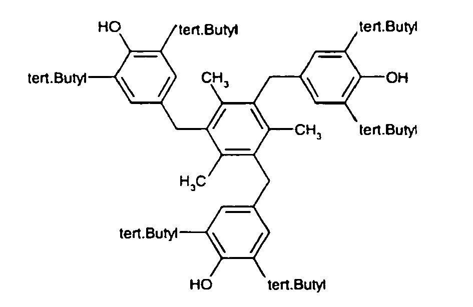
- Tetrakis-[methylen-3(3',5'-di-t-butyl-4'-hydroxyphenyl)propionat]methan (CAS 6683-19-8);
- Verbindung der Formel 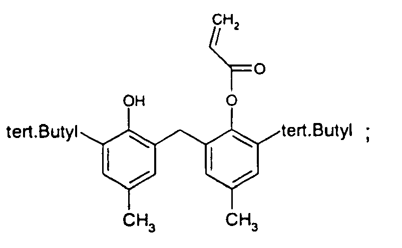
- Verbindung der Formel 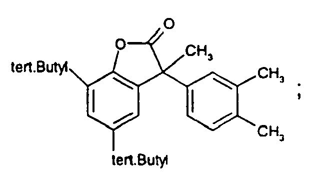
- Vanillin;
- Ubichinon;
- Ferulasäure und -Derivate;
- Rutinsäure und -Derivate;
- Urocaninsäure und -Derivate; und
- Propolis.

Vorzugsweise werden folgende Mischungen aus Antioxidantien und organischen UV-Filtern verwendet:
- Mischungen aus Methylen-bis-benzotriazolyltetramethylbutylphenol, Octyltriazon, Titandioxid und Tocopherol,
- Mischungen aus 2,2'-Methylen-bis-[6-(2H-benztriazol-2-yl)-4-methyl-phenol, Octyltriazon, Trisresorcinyltriazin und Vitamin E
- Mischungen aus Methylen-bis-benzotriazolyltetramethylbutylphenol, Octyltriazon, Verbindung der Formel (103) 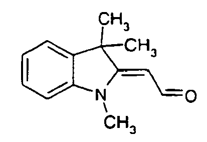 ("Fischeraldehyd") und Verbindung der Formel

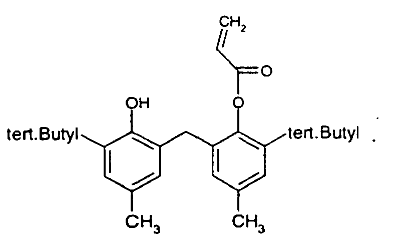

Composite sind durch Einschmelzen von schmelzbaren kosmetischen, pflanzlichen und pharmazeutischen Wirkstoffen in organische UV-Filter erhältlich.

Generell können mikronisierte UV-Filter als Träger von hochaktiven Stoffen, insbesondere kosmetischen und/oder pharmazeutischen Wirksubstanzen genutzt werden. Der Vorteil derartiger Composites liegt in der Möglichkeit, den bzw. die aktiven Stoffe aus dem Festkörper freizusetzen (slow release). Eine langsame Freisetzung garantiert über die gesamte Nutzungsdauer der UV-Pigmente auch die gleichmässige Wirksamkeit von hochaktiven Wirkstoffen, wie z.B. Entzündungshemmern, Pflegewirkstoffen oder Spurenelementen, wie z.B. Zn²⁺ oder Mg²⁺.

Als beispielhaft einsetzbare Wirkstoffe seien erwähnt:
- Wirkstoffe zur antimikrobiellen Ausrüstung und gleichzeitigen antünflamatorischen Wirkung, wie z.B. Triclosan oder Diclosan;
- entzündungshemmende Wirkstoffe, wie z.B. Farnesol, Panthenol oder Avocadoöl;
- Wirkstoffe mit Deo- bzw. Antiperspirantwirkung, wie z.B. Zn-Ricinoleate und Alkylcitrate,
- Undecylensäure und deren Derivate (z.B. Diethanolamide)
- Zinkundecylat;
- Pyrithione, wie z.B. Natriumpyrithion;
- eingeschmolzene Riechstoffe oder Riechstoffgemische, wie z.B. Menthol, Geraniol usw., die diesen Mikropigmenten und den Formulierungen, die diese enthalten, einen permanenten und gleichintensiven Geruch verleihen.

Zur Herstellung der Mikropigmentgemische können alle bekannten Verfahren, die für die Herstellung von Mikropartikeln geeignet sind, genutzt werden, wie z.B.:
- Nassmahlung mit einem hartem Mahlkörper wie z.B. Zirkoniumsilikat und einem Schutztensid oder einem Schutzpolymeren in Wasser oder einem geeigneten organischen Lösungsmittel;
- Sprühtrocknung aus einem geeigneten Lösungsmittel, wie z.B. wässrige oder organische Lösungsmittel enthaltende Suspensionen oder echte Lösungen in Wasser, Ethanol, Dichlorethan, Toluol, N.Methylpyrrolidon u.a..
- Durch Entspannung von superkritischen Flüssigkeiten (z.B. CO₂) nach dem RESS-Prozess (Rapid Expansion of Supercritical Solutions), in denen der oder die UV-Filter gelöst ist/sind oder Entspannung von flüssigem Kohlendioxid gemeinsam mit einer Lösung eines oder mehrerer UV-Filter in einem geeigneten organischen Lösungsmittel;
- durch Umfällen aus geeigneten Lösungsmitteln, einschließlich superkritischen Flüssigkeiten (GASR-Prozeß = Gas Anti-Solvent Recrystallisation / PCA-Prozess = Precipitation with Compressed Antisolvents).

Als Mahlapparate zur Herstellung der erfindungsgemässen mikronisierten organischen UV-Absorber können z.B. eine Düsen-, Kugel-, Vibrations- oder Hammermühle, vorzugsweise eine Hochgeschwindigkeits-Rührmühle verwendet werden. Die Mahlung erfolgt vorzugsweise mit einer Mahlhilfe, wie z.B. einem alkylierten Vinylpyrrolidon-Polymer, einem Vinylpyrrolidon-Vinylacetat-Copolymer, einem Acylglutamat, einem Alkylpolyglucosid, Ceteareth-25 oder insbesondere einem Phospholipid.

Die so erhaltenen Mikropigmente bzw. Gemische aus Mikropigmenten haben gewöhnlich eine mittlere Partikelgrösse von 0,02 bis 2, vorzugsweise 0,05 bis 1,5, und ganz besonders von 0,1 bis 1,0 µm.

Auf Grund ihrer Lipophilität lassen sie sich alleine oder zusammen mit anderen löslichen organischen UV-Absorbern nach bekannten Methoden gut in öl- und fetthaltige kosmetische Formulierungen, wie z.B. Öle, O/W- oder W/O-Emulsionen, Fettstifte oder Gele einarbeiten.

Überraschend erhält man Formulierungen mit gleicher oder verbesserter Schutzwirkung bei Verwendung von weniger oder gar keinen löslichen UV-AbsorberN.

Einen weiteren Erfindungsgegenstand bildet ein kosmetische Formulierung, enthaltend ein Gemisch aus Mikropigmenten, gegebenenfalls einen oder mehrere Antioxidantien und/oder anorganische Pigmente und/oder eine kationische bzw. anionische Verbindung, sowie kosmetisch verträgliche Träger- oder Hilfsstoffe.

Erfindungsgemässe kosmetische Formulierungen beinhalten die verschiedensten kosmetischen Mittel. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Diese aufgezählten Endformulierungen können in den verschiedensten Darreichungsformen vorliegen, wie z.B.
- in Form von flüssige Zubereitungen als einer W/O- O/W-, O/W/O-, W/O/W-. PIT- und aller Arten von Mikroemulsionen
- in Form eines Gels,
- in Form eines Öls, einer Creme, Milch oder Lotion,
- in Form eines Pulvers, eines Lacks, einer Tablette oder Make-Ups,
- in Form eines Stiftes,
- in Form eines Sprays (Spray mit Treibgas oder Pumpspray) oder eines Aerosols,
- in Form eines eines Schaumes, oder
- in Form einer Paste.

Vorteilhaft können die erfindungsgemässen kosmetischen Formulierungen weitere Substanzen enthalten, die UV-Strahlung im UVB-Bereich absorbieren. Die Gesamtmenge der Filtersubstanzen sind dabei 0,1 bis 30, vorzugsweise 0,5 bis 10 Gew.-%, insbesondere 1 bis 6 Gew.-%, bezogen auf das Gesamtgewicht der Zusammensetzung.

Insbesondere kommen als zusätzliche UVB-Filter öllösliche, nicht mikronisierte Verbindungen in Betracht, wie z.B. organische UV-Absorber aus der Klasse der p-Aminobenzoesäurederivate, Salicylsäurederivate, Benzophenonderivate, Dibenzoylmethanderivate, Diphenylacrylatderivate, Benzofuranderivate, polymere UV-Absorber, enthaltend eine oder mehrere silizium-organsiche Reste, Zimtsäurederivate, Campherderivate, Trianilino-s-Triazinderivate, Phenylbenzimidazolsulfonsäure und deren Salze, Menthyl-Anthranilate, Benzotriazolderivate, und/oder ein anorganisches Mikropigment ausgewählt aus mit Aluminiumoxid oder Siliciumdioxid umhülltem TiO₂, Zinkoxid oder Mica.
- Beispielhafte Verbindungen für p-Aminobenzoesäurederivate: 4-Aminobenzoesäure (PABA); Ethyldihydroxypropyl-PABA der Formel 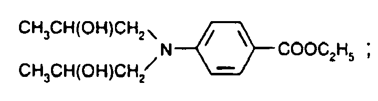 PEG-25-PABA der Formel 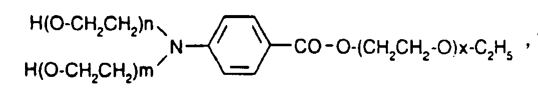 worin m, n und x dieselbe
   Bedeutung haben und je höchstens 25 bedeuten;
   Octyldimethyl PABA der Formel 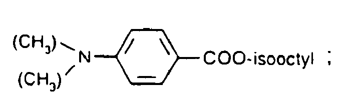 oder
   Glycylaminobenzoat der Formel 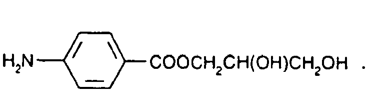
- Beispielhafte Verbindungen für Salicylsäurederivate:
   Homomenthylsalicylat der Formel 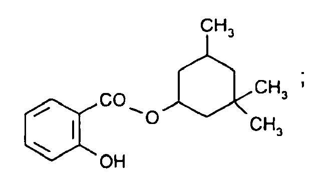
   Triethanolaminsalicylat der Formel 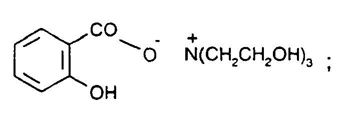 Amyl-p-dimethylaminobenzoat der Formel (10) 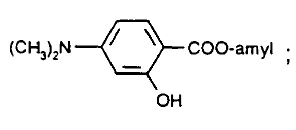 Octylsalicylat der Formel 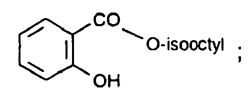 oder 4-Isopropylbenzylsalicylat der Formel 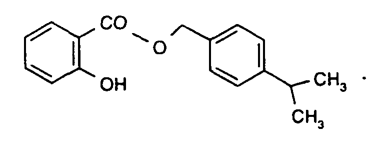
- Beispielhafte Verbindungen für Benzophenonderivate:
   Benzophenon-3-(2-hydroxy-4-methoxybenzophenon), Benzophenon-4-(2-hydroxy-4-methoxybenzophenon-5-sulfonsäure) oder Benzophenon-8-(2,2'-dihydroxy-4-methoxybenzophenon).
- Beispielhafte Verbindungen für Dibenzoylmethanderivate:
   Butylmethoxydibenzoylmethan-[1-(4-tert.-butyl)-3-(4-methoxyphenyl)propan-1,3-dion].
- Beispielhafte Verbindungen für Diphenylacrylatderivate:
   Octocrylen-(2-ethylhexyl-2-cyano-3,3'-diphenylacrylat) oder etocrylen-(ethyl-2-cyano-3,3'-diphenylacrylat).
- Beispielhafte Verbindungen für Benzofuranderivate:
   3-(Benzofuranyl)-2-cyanoacrylat, 2-(2-Benzofuranyl)-5-tert.-butylbenzoxazol oder 2-(p-Aminophenyl)benzofuran und insbesondere die Verbindung der Formel 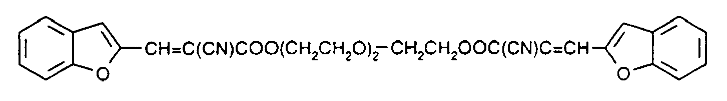 oder 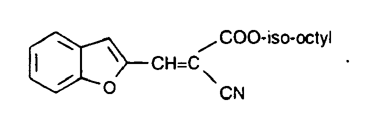
- Beispielhafte Verbindungen für polymere UV-Absorber, die eine oder mehrere siliziumorganische Reste enthalten:
   Benzylidenmalonatderivat, insbesondere die Verbindung der Formel 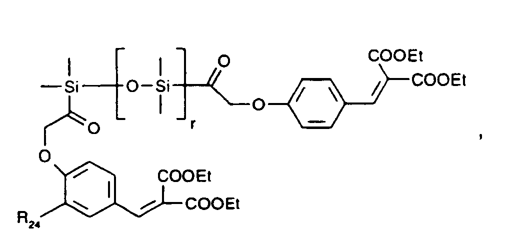 worin
      - R₂₄: Wasserstoff oder Methoxy und
      - r: ungefähr 7; die Verbindung der Formel 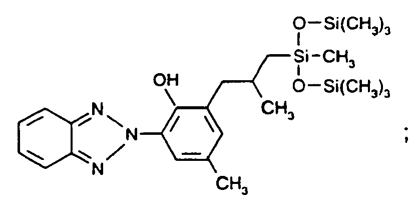 oder 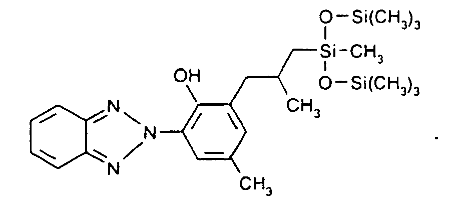
- Beispielhafte Verbindungen für Zimtsäureester:
   Octylmethoxycinnamat (4-Methoxyzimtsäure-2-ethylhexylester), Diethanolaminmethoxycinnamat (Diethanolaminsalz der 4-Methoxyzimtsäure), lsoamyl-p-methoxycinnamat (4-Ethoxyzimtsäure-2-isoamylester), 2,5-Diisopropylmethylcinnamat oder ein Zimtsäureamidoderivat.
- Beispielhafte Verbindungen für Campherderivate:
   4-Methyl-benzylidencampher [3-(4'-Methyl)benzyliden-bornan-2-on], 3-Benzyldencampher (3-Benzyliden-bornan-2-on), Polyacrylamidomethylbenzylidencampher {N-[2(und 4)-2-oxyborn-3-yliden-methyl)benzyl]acrylamidpolymer}, Trimonium-benzyliden-camphersulfat-[3-(4'-trimethylammonium)-benzyliden-bornan-2-on-methylsulfat], Terephthalydendicampher-Sulfonsäure {3,3'-(1,4-Phenylendimethin)-bis-(7,7-dimethyl-2-oxo-bicyclo-[2.2.1]heptan-1-methansulfonsäure} oder deren Salze, oder Benzylidencampher-Sulfonsäure [3-(4'-sulfo)benzylidenbornan-2-on] oder deren Salze.
- Beispielhafte Verbindungen für Trianilino-s-triazinderivate:
   Octyltriazin-[2,4,6-trianilino-(p-carbo-2'-ethyl-1'-oxy)-1,3,5-triazin, sowie die in der US-A-5,332,568, US-A-5,252,323, WO 93/17002 und WO 97/03642 und EP-A-0,517,104 beschriebenen Trianilino-s-triazinderivate.
- Beispielhafte Verbindungen für Benzotriazole:
   2-(2-Hydroxy-5-methyl-phenyl)benzotriazol.

Die folgenden Beispiele dienen zur Erläuterung der Erfindung, ohne sie auf diese zu beschränken. Die kosmetischen Wirksubstanzen sind vorrangig mit ihrer INCI-Bezeichnung (INCI = International Norm of Cosmetical Ingredients) angegeben.

### Beispiel 1

50 Teile Methylene Bis-benzotriazolyl Tetramethylbutylphenol und 50 Teile Octyl Triazone werden zusammen mit einem Mahlkörper aus Zirkoniumsilikat-Sand, einem Schutztensid (Alkyl Polyglucoside) und Wasser in einer Peri-Mühle zu einem Misch-Mikropigment mit einem d₆₀ von 190 nm vermahlen, Nach Abtrennen des Mahlkörpers kann die Suspension des Misch-Mikropigments zur Herstellung von Sonnenschutzformulierungen verwendet werden.

### Beispiel 2:

32 Teile Octyl Triazone, 1 Teil Cetyltrimethylammoniumbromid und 66 Teile Methylene Bis-benzotriazolyl Tetramethylbutylphenol werden homogen zusammengeschmolzen. Es wird rasch auf Raumtemperatur abgekühlt und die erstarrte Schmelze mechanisch (schlagmühle) zerkleinert. Dieses so erhaltene Pulver wird in Wasser angeschlämmt, Decyl Glycoside zugefügt und zusammen mit einem Mahlhilfsmittel ('schwerer Sand') bis auf eine Teichengröße von d₅₀ 200 nm Durchmesser mikronisiert. Nach Abtrennung des Mahlhilfsmittels erhält man eine wässrige Suspension des mikronisierten UV-Absorbercomposites. Diese Suspension wird mit Zitronensäure leicht sauer eingestellt und kann zur Herstellung von kosmetischen und pharmazeutischen Formulierungen verwendet werden.

### Beispiel 4:

In 75 Teilen geschmolzenem Methylene Bis-benzotriazolyl Tetramethylbutylphenol werden 25 Teile Dioctyl Butamido Triazone gelöst. Es wird rasch abgekühlt, das Gemisch mechanisch zu feinem Pulver zerkleinert und danach mit einem Mahlkörper aus Zirkoniumsilikat-Sand, einem Schutztensid (Phospholipid) und Wasser zu einem Mikropigment mit einem d₅₀ 300 nm vermahlen. Die vom Mahlkörper abgetrennte Suspension des Mikropigmentes wird zur Herstellung von Sonnenschutzformulierungen verwendet.

### Beispiel 5:

In 70 Teilen geschmolzenes Methylene Bis-benzotriazolyl Tetramethylbutylphenol werden mit 24 Teilen Octyl Triazone, 5 Teilen Titanium Dioxide und einem Teil Tocopherol vermischt. Es wird rasch abgekühlt, das Gemisch mechanisch zu feinem Pulver zerkleinert und danach mit einem Mahlkörper aus Zirkoniumsilikat-Sand, einem Schutztensid (Alkyl Polyglucosid) und Wasser zu einem Mikropigment vermahlen. Die vom Mahlkörper abgetrennte Suspension des Mikropigmentes wird zur Herstellung von Sonnenschutzformulierungen verwendet.

In den folgenden Beispielen 6 bis 11 werden analog zu den Beispielen 1 und 2 Suspensionen von Mikrocomposites folgender Zusammensetzungen hergestellt:

### Beispiel 6:

60 Teile 2,2'-Methylen-bis-[6-(2H-benztriazol-2-yl)-4-methyl-phenol,
20 Teile Octyl triazone, 19 TeileTris Resorcinyl Triazin und 1 Teil Vitamin E, eingestellt auf pH 6,5 mit Zitronensäure.

### Beispiel 7:

60 Teile 2,2'-Methylen-bis-[6-(2H-benztriazol-2-yl)-4-methyl-phenol,
20 Teile Octyl Triazone und 20 Teile der Verbindung der Formel (101)

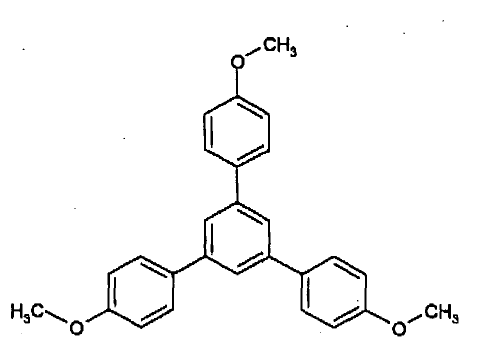

### Beispiel 8:

59 Teile 2,2'-Methylen-bis-[6-(2H-benztriazol-2-yl)-4-methyl-phenol,
20 Teile Octyl Triazone,
20 Teile der Verbindung der Formel (102) 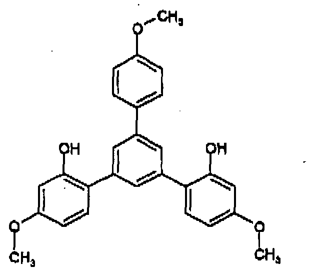 und eingestellt auf pH 6,5 mit Zitronensäure.

### Beispiel 10:

80 Teile Methylene Bis-benzotriazolyl Tetramethylbutylphenol, und
20 Teile der Verbindung der Formel (104)

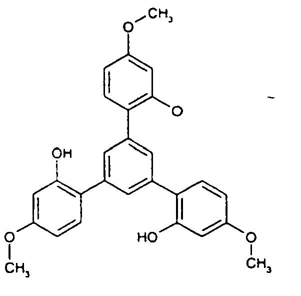

### Beispiel 11:

50 Teile Methylene Bis-benzotriazolyl Tetramethylbutylphenol,
10 Teile Dioctyl Butamido Triazone (Mahlung bei pH < 5, eingestellt auf pH 6,5 mit Zitronensäure) und
20 Teile der Verbindung der Formel (102).

### Beispiel 12: O/W-Sonnenschutz-Lotion

| | | % | |
|---|---|---|---|
| A | Polyglyceryl-3 Methylglucose Distearate | 2,0 | |
| | Decyl Oleate | 5,7 | |
| | Isopropyl Palmitate | 6,0 | |
| | Caprylic/Capric Triglycerid | 7,5 | |
| | | | |
| B | Glycerin | 3,0 | |
| | Phenonip | 0,5 | |
| | Wasser | 69,3 | |
| | | | |
| C | Carbomer | 0,2 | |
| | Isopropyl Palmitate | 0,8 | |
| | | | |
| D | Mikropigment aus Beispiel 2 | 5,0 | |
| | | | |
| E | NaOH (10%ig) | nach Bedarf | |
| | | | |

### Beispiel 13: O/W-Emulsion

| | % |
|---|---|
| Potassium Cetyl Phosphate | 2,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Glyceryl Stearate | 3,00 |
| Cetyl Alcohol | 1,00 |
| Phenoxyethanol&Parabens | 1,00 |
| Octyl Methoxycinnamate | 5,00 |
| Dimethicone | 0,10 |
| Deionisiertes Wasser | 64,15 |
| Carbomer (Carbopol 981) | 0,10 |
| Glycerin | 3,00 |
| NaOH (10%ig) | 1,00 |
| Mikropigment aus Beispiel 1 | 4,00 |

### Beispiel 15: O/W-Emulsion:

| | % |
|---|---|
| Isopropylmyristate&Trilaureth-4 Phosphate | 5,00 |
| Tricontanyl PVP | 1,00 |
| Caprylic/Capric Triglyceride | 5,00 |
| Cetearyl Isononanoate | 2,00 |
| C12-15 Alkyl Benzoate | 5,00 |
| Glyceryl Stearate | 2,00 |
| Cetyl Alcohol | 1,00 |
| Phenoxyethanol&Parabens | 1,00 |
| Octyl Methoxycinnamate | 5,00 |
| Dimethicone | 0,10 |
| | |
| Deionisiertes Wasser | 66,30 |
| Carbomer (Carbopol 981) | 0,10 |
| Glycerin | 3,00 |
| NaOH (10%ig) | 0,50 |
| | |
| Mikropigment aus Beispiel 4 | 4,00 |

### Beispiel 16: O/W-Emulsion

| | % |
|---|---|
| Sodium Stearyl Lactate Tricontanyl PVP | 1,50 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Glyceryl Stearate | 3,50 |
| Cetyl Alcohol | 2,00 |
| Phenoxyethanol&Parabens | 1,00 |
| Octyl Methoxycinnamate | 5,00 |
| Dimethicone | 0,20 |
| | |
| Deionisiertes Wasser | 63,60 |
| Carbomer (Carbopol 981) | 0,10 |
| Glycerin | 3,00 |
| NaOH (10%ig) | 0,10 |
| | |
| Mikropigment aus Beispiel 6 | 4,00 |

### Beispiel 18: O/W-Emulsion

| | % |
|---|---|
| Lauryl Glucoside & Polyglyceryl-2 Dihydroxystearate & Glycerin | 3,00 |
| Tricontanyl PVP | 1,00 |
| Caprylic/Capric Triglyceride | 4,00 |
| Cetearyl Isononanoate | 4,00 |
| C12-15 Alkyl Benzoate | 5,00 |
| Glyceryl Stearate | 2,00 |
| Cetyl Alcohol | 3,00 |
| Phenoxyethanol & Parabens | 1,00 |
| Cetyl Methoxycinnamate | 5,00 |
| Dimethicone | 0,20 |
| | |
| Deionisiertes Wasser | 64,49 |
| Carbomer (Carbopol 981) | 0,10 |
| Glycerin | 3,00 |
| NaOH (10%ig) | 0,21 |
| | |
| Mikropigment aus Beispiel 8 | 4,00 |

### Beispiel 19: O/W-Emulsion:

| | % |
|---|---|
| Cetaryl Glucoside & Cetearyl Alcohole | 4,50 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octyl triazone | 3,00 |
| 4-Methylbenzylidene camphor | 3,00 |
| Dimethicone | 0,20 |
| | |
| Deionisiertes Wasser | 64,65 |
| Steareth-10 Allyl Ether(Acrylates Copolymer | 5,00 |
| Glycerin | 3,00 |
| NaOH (10%ig) | 1,00 |
| | |
| Mikropigment aus Beispiel 2 | 4,00 |

### Beispiel 20: O/W-Emulsion

| | % |
|---|---|
| Cetearyl Glucoside | 5,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octocrylene | 3,00 |
| Octyl Methoxycinnamate | 4,00 |
| Dimethicone | 0,20 |
| | |
| Deionisiertes Wasser | 63,15 |
| Carbomer (Carbopol 981) | 0,50 |
| Glycerin | 3,00 |
| NaOH (10%ig) | 0,15 |
| | |
| Mikropigment aus Beispiel 2 | 4,00 |

### Beispiel 22: O/W-Emulsion:

| | % |
|---|---|
| Palmitic Acid & Stearic Acid | 1,80 |
| Glyceryl Stearate SE | 3,00 |
| Tricontanyl PVP | 1,00 |
| Caprylic/Capric Triglyceride | 5,00 |
| Cetearyl Isononanoate | 5.00 |
| C12-15 Alkyl Benzoate | 5,00 |
| Glyceryl Stearate | 0,50 |
| Phenoxyethanol & Parabens | 1,00 |
| Octyl dimethyl PABA | 5,00 |
| Dimethicone | 0,10 |
| Deionisiertes Wasser | 64,15 |
| Carbomer (Carbopol 981) | 0,10 |
| Glycerin | 3,00 |
| NaOH (10%ig) | 0,50 |
| Mikropigment aus Beispiel 1 | 4,00 |

### Beispiel 24: O/W-Emulsion:

| | % |
|---|---|
| Steareth-2 | 2,50 |
| Steareth-21 | 1,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Cetyl Alcohol | 1,00 |
| Phenoxyethanol&Parabens | 1,00 |
| Methyl anthranilate | 3,00 |
| Octyl Methoxycinnamate | 4,00 |
| Dimethicone | 0,10 |
| Deionisiertes Wasser | 63,95 |
| Carbomer (Carbopol 981) | 0,20 |
| Glycerin | 3,00 |
| NaOH (10%ig) | 0,25 |
| | |
| Mikropigment aus Beispiel 4 | 4,00 |

### Beispiel 26: O/W-Emulsion

| | % |
|---|---|
| Octyldecyl Phosphate | 3,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octyl methoxycinnamate | 5,00 |
| Dimethicone | 0,10 |
| | |
| Deionisiertes Wasser | 64,50 |
| Sodium Cocoyl Glutamate | 0,60 |
| Fteareth-10 Allyl Ether/ Acrylates Copolymer | 0,50 |
| Glycerin | 3,00 |
| NaOH (10%ig) | 2,30 |
| Mikropigment aus Beispiel 4 | 4,00 |

### Beispiel 27: O/W-Emulsion:

| | % |
|---|---|
| Polyglyceryl-3 Methyl Glucose Distearate | 2,00 |
| Tricontanyl PVP | 1,00 |
| Tocopherol&Ascorbyl Palmitate&Ascorbic Acid&Citric Acid&PEG-8 | 0,05 |
| Decyl Oleate | 4,50 |
| Isopropyl Palmitate | 6,00 |
| Caprylic/Capric Triglyceride | 5,00 |
| Glyceryl Stearate | 1,00 |
| Cetearyl Alcohol | 1,00 |
| 2-[(2,4-methoxy)-phenyl]-4,6-bis-[(2-hydroxy-4-methoxy)-phenyl]-(1,3,5)-triazine | 2,00 |
| Octyl Methoxycinnamate | 3,00 |
| deionisiertes Wasser | 63,12 |
| Phenoxyethanol&Parabens | 0,80 |
| Propylene Glycol | 3,00 |
| Carbomer (Carbopol 981) | 0,20 |
| NaOH (10%ig) | 0,33 |
| Scleroglucan | 1,00 |
| Mikropigment aus Beispiel 2 | 3,00 |
| Titanium Dioxide | 3,00 |

### Beispiel 28: O/W-Emulsion

| | % |
|---|---|
| Methyl Glucose Sequistearate | 2,50 |
| Tricontanyl PVP | 1,00 |
| Tocopherol&Ascorbyl Palmitate&Ascorbic Acid&Citric Acid&PEG-8 | 0,05 |
| Decyl Oleate | 4,00 |
| Isopropyl Palmitate | 6,00 |
| Caprylic/Capric Triglyceride | 5,00 |
| Glyceryl Stearate | 1,00 |
| Cetearyl Alcohol | 1,00 |
| 2-[(2,4-methoxy)-phenyl]-4,6-bis-[(2-hydroxy-4-methoxy)-phenyl]-(1,3,5)-triazine | 2,00 |
| Octyl Methoxycinnamate | 5,00 |
| | |
| deionisiertes Wasser | 63,12 |
| Phenoxyethanol&Parabens | 0,80 |
| Carbomer (Carbopol 981) | 0,20 |
| Glycerin | 3,00 |
| NaOH (10%ig) | 0,33 |
| Scleroglucan | 1,00 |
| Mikropigment aus Beispiel 1 | 4,00 |

### Beispiel 29: Lippenpfleqemittel

| | % |
|---|---|
| Glycerin | 10,00 |
| PEG-45&Dodecyl Glycerol Copolymer | 1,50 |
| Quaternium-18 Bentonit | 2,00 |
| Microkristallines Wachs | 2,00 |
| Bienenwachs | 2,00 |
| Glyceryl Stearate SE | 53,00 |
| Pentaerythrithil Stearat&Caprate&Caprylat Adipate | 4,00 |
| Castor Oil | 4,00 |
| Methylene Bis-benzotriazolyl Tetramethylbutylphenol | 5,00 |
| Mikropigment Beispiel 2 | 5,00 |
| Titanium Dioxide | 5,00 |
| Zink Oxide | 5,00 |
| Octyl Methoxycinnamate | 4,00 |
| Eucerinum anhydricum | ad 100 |

### Beispiel 30: W/O-Emulsion

| | % |
|---|---|
| PEG-30 Dipolyhydroxystearate | 2,00 |
| Isostearyl Alcohol | 20,00 |
| Isostearic Acid | 10,00 |
| Octyl Triazone | 3,00 |
| | |
| deionisiertes Wasser | 58,75 |
| Glycerin | 5,00 |
| Methylparaben | 0,17 |
| Propylparaben | 0,03 |
| MgSO₄x7H₂O | 0,75 |
| | |
| Mikropigment aus Beispiel 2 | 4,00 |

### Beispiel 31: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | Octyl Methoxycinnamate | 5,0 |
| | | |
| B | Glycerol | 3,0 |
| | Phenonip | 0,5 |
| | deion. Wasser | 62,9 |
| | | |
| C | Carbomer 141 | 0,2 |
| | Isopropyl Palmitate | 0,8 |
| | | |
| D | 50%ige Suspension aus Beispiel 8 | 8,0 |
| | | |
| E | NaOH (10%) | nach Bedarf |

### Beispiel 32: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | | |
| B | Glycerol | 3,0 |
| | Phenonip | 0,5 |
| | deionis. Wasser | 62,9 |
| | | |
| C | Carbomer 141 | 0,2 |
| | Isopropyl Palmitate | 0,8 |
| | | |
| D | Suspension aus Beispiel 2 | 6,0 |
| | | |
| E | NaOH (10%) | nach Bedarf |

### Beispiel 33: (O/W-Emulsion)

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | Octyl Triazone | 2,0 |
| | | |
| B | Glycerol | 3,0 |
| | Phenonip | 0,5 |
| | Wasser | 62,3 |
| | | |
| C | Carbomer 141 | 0,2 |
| | Isopropyl Palmitate | 0,8 |
| | | |
| D | 2,2'-Methylene-bis-(6-(2H-benzotriazol-2-yl)-4-(1,1,3,3-tetrametylbutyl)phenol-Mikropigment-Suspension (50%) | 8,0 |
| | Octyl Triazone-Mikropigment-Suspension (50%ig) | 4,0 |
| | | |
| E | NaOH (10%) | nach Bedarf |

### Beispiel 34: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Octyl Triazone | 2,0 |
| | Caprylic/Capric Triglyceride | 6,5 |
| | | |
| B | Glycerol | 3,0 |
| | Phenonip | 0,5 |
| | Wasser | 68.3 |
| | | |
| C | Carbomer 141 | 0,2 |
| | Isopropyl Palmitate | 0,8 |
| | | |
| D | Mikropigment aus Beispiel 2 | 6,0 |
| | | |
| E | NaOH (10%) | nach Bedarf |

### Beispiel 36: W/O -Emulsion

| | % |
|---|---|
| Methoxy PEG-22/Dodecyl Glycol Copolymer (Elfacos E 200^{®}) | 3,00 |
| PEG-22/ Dodecyl Glycol Copolymer (Elfacos ST 37^{®}) | 3,00 |
| Hydroxyoctacosanyl Hydroxystearate (Elfacos C 26^{®}) | 3,00 |
| Octyl Stearate | 15,00 |
| Coco Glycerides | 2,00 |
| Mineral Oil | 3,00 |
| Phenoxyethanol&Parabens | 1,00 |
| 4-Methylbenzylidene Camphor | 3,00 |
| Dioctyl Butamido Triazone | 3,00 |
| Dimethicone | 0,20 |
| Wasser | 53,00 |
| Phenylbenzimidazol Sulphonsäure | 3,00 |
| Magnesium Sulphate (MgSO₄ x 7 H₂O) | 0,80 |
| Propylene Glycol | 4,00 |
| Mikropigment aus Beispiel 5 | 3,00 |

### Beispiel 37: W/O -Emulsion

| | % |
|---|---|
| Polyglyceryl-2 Dipolyhydroxystearate (Dehymuls PGPH^{®}) | 2,00 |
| PEG-30 Dipolyhydroxystearate (Arlacel P 135^{®}) | 2,00 |
| Hydroxyoctacosanyl Hydroxystearate (Elfacos C 26^{®}) | 2,00 |
| Zink Stearate | 1,00 |
| Octyl Stearate | 15,00 |
| Coco Glycerides | 2,00 |
| Mineral Oil | 3,00 |
| Phenoxyethanol & Parabens | 1,00 |
| 2,4-Bis-{[4-(2-Ethyl-hexyloxy)-2-hydroxy]-phenyl}-6-(4-methoxyphenyl)1,3,5)-triazine | 2,00 |
| Octyl Salicylate | 3,00 |
| Dimethicone | 0,20 |
| Wasser | 56,70 |
| Magnesium Sulphate (MgSO₄ x 7 H₂O) | 1,00 |
| Propylene Glycol | 4,00 |
| Mikropigment aus Beispiel 6 | 5,00 |

### Beispiel 39: W/O -Emulsion

| | % |
|---|---|
| Polyglyceryl-2-Dipolyhydroxystearate (Dehymuls PGPH^{®}) | 3,00 |
| Glyceryl Oleate (Monomuls 90-0 18^{®}) | 1,00 |
| Caprylic / Capric Triglyceride | 6,00 |
| Octyldodecanol | 6,00 |
| Cetearyl Isononaoate | 5,00 |
| Octyl Methoxycinnamate | 3,00 |
| Tocopheryl Acetate | 1,00 |
| Cera alba | 1,20 |
| Glycerin (86 %) | 5,00 |
| Phenonip | 0,50 |
| Mikropigment aus Beispiel 10 | 5,00 |
| Wasser | ad 100 |

### Beispiel 40: O/W-Emulsion

| | % |
|---|---|
| Tego Care CG 90 (Goldschmidt AG) | 6,00 |
| Cetearyl Alcohol | 1,50 |
| Glycerylstearat | 0,50 |
| Octyldecanol | 7,00 |
| Capric/Caprylic Triglyceride | 5,00 |
| Cetearylisononaoat | 6,00 |
| Octyl Methoxycinnamate | 3,00 |
| Deionisiertes Wasser | 51,14 |
| Carbomer | 0,20 |
| NaOH (45%ig) | 1,13 |
| Glycerin | 5,00 |
| Methylparaben | 0,17 |
| Propylparaben | 0,03 |
| Terephthalyden-dibornan-sulphonsäure | 1,50 |
| Mikropigment aus Beispiel 5 (50%ige Suspension) | 12,00 |

### Beispiel 42: O/W/O-Emulsion

| | % |
|---|---|
| Polyglyceryl-2-polyhydroxystearat | 5,0 |
| Mineralöl | 12,5 |
| Stearinsäure | 2,0 |
| Cetearylisononaoat | 12,5 |
| Methylbenzylidene Camphor | 2,0 |
| Homosalate | 2,0 |
| Deionisiertes Wasser | ad 100,0 |
| Carbomer | 0,2 |
| Konservierungsmittel | nach Bedarf |
| NaOH | nach Bedarf |
| Mikropigment aus Beispiel 2 (50%ige Suspension) | 8,0 |

### Beispiel 43: O/W -Emulsion

| | % |
|---|---|
| Glycerinstearat/Polyetylenglycol(MG100)-stearat | 3,0 |
| Cetyl-/Stearylalkohol-20EO (Eumulgin B 2) | 1,0 |
| Cetyl-/Stearylalkohol (Lanette O) | 2,0 |
| Capryl-/Caprin-triglycerid (Myritol 318) | 4,0 |
| Dicaprylether | 6,0 |
| Mineralöl und Quarternium-18 Hectorite | 3,0 |
| Glycerinstearat, Cetyl-/Stearylalkohol, Cetylpalmitat, Kokosglyceride (Cutina CBS) | 2,0 |
| 4-Methylbenzylidene Camphor | 1,0 |
| Octyl Triazone | 2,0 |
| Deionisiertes Wasser | ad 100,0 |
| Glycerin, 85%ig | 3,0 |
| Konservierungsmittel | nach Bedarf |
| Magnesiumaluminiumsilikat (Vegum Ultra) | 0,3 |
| NaOH | nach Bedarf |
| Mikropigment aus Beispiel 2 (50%ige Suspension) | 10,0 |

## Patentansprüche

1. Verwendung von Mischungen aus mikronisierten organischen UV-Filtern ausgewählt aus
- Mischungen aus Octyltriazon und Methylene Bis benzotriazolyltetramethylbutylphenol;
- Mischungen aus 2-[(2,4-methoxy)-pheny]-4,6-bis-[(2-hydroxy-4-methoxy)-phenyl]-(1,3,5)-triazin und Methylen-bis-benzotriazolyltetramethylbutylphenol;
- Mischungen aus Methylen-bis-benzotriazolyltetramethylbutylphenol und Dioctybutamidotriazon;
- Mischungen aus 2,2'-Methylen-bis-[6-(2H-benztriazol-2-yl)-4-methyl-phenol, Octyltriazon und der Verbindung der Formel (36) 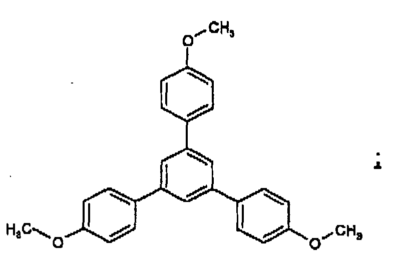
- Mischungen aus 2,2'-Methylen-bis-[6-(2H-benztriazol-2-yl)-4-methyl-phenol, Octyltriazon und der Verbindung der Formel (37) 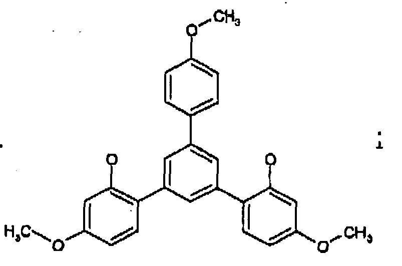
- Mischungen aus Methylen-bis-benzotriazolyltetramethylbutylphenol und der Verbindung der Formel (39) 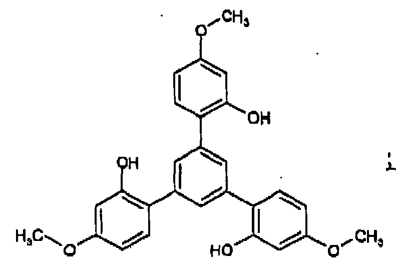 und
- Mischungen aus Methylen-bis-benzotriazolyltetramethylbutylphenol, Dioctylbutamidotriazon und der Verbindung der Formel (37) 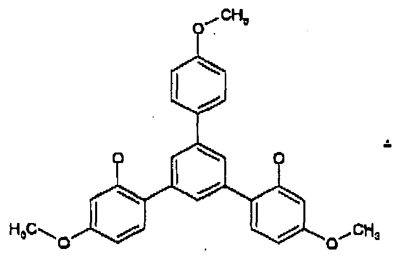
zur Herstellung eines Medikaments zum Schützen der menschlichen und tierischen Haut und der Haare vor der schädigenden Einwirkung von UV-Strahlung, wobei die mikronisierten organischen UV-Filter eine Partikelgrösse von 0,02 bis 2 µm aufweisen.

2. Verwendung nach Anspruch 1. **dadurch gekennzeichnet dass** man
- Mischungen aus Methylene_Bis-benzotriazolyltetramethylbutylphenol und Octyltriazon:
- Mischungen aus 2-[(2,4-methoxy)-phenyl]-4,6-bis-[(2-hydroxy-4-methoxy)-phenyl]-(1,3,5)-triazin und Methylen-bis-benzotriazolyltetramethylbutylphenol:
- Mischungen aus Methylen-bis-benzotriazolyltetramethylbutylphenol und Dioctybutamidotriazon; und
- Mischungen aus Methylen-bis-benzotriazolyltetramethylbutylphenol und der Verbindung der Formel (39) 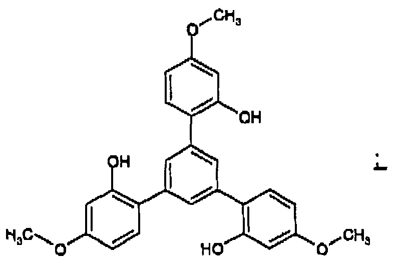
verwendete werden.

3. Verwendung nach Anspruch 1 oder 2. **dadurch gekennzeichnet dass** man Mischungen aus Methylene Bis-benzotriazolyltetramethylbutylphenol und Octyltriazon verwendet.

4. Verwendung nach Anspruch 1 ode 2, **dadurch gekennzeichnet dass** man Mischungen aus Methylen-bis-benzotriazolyltetramethylbuylhenol und Dioctybutamidotriazon verwendet.

5. Verwendung nach einem der Ansprüche 1 bis 4, **dadurch gekennzeichnet, dass** zusätzlich ein anorganisches Pigment hinzugemischt wird.

6. Verwendung nach einem der Ansprüche 1 bis 4, **dadurch gekennzeichnet, dass** zusätzlich ein Antioxidans hinzugemischt wird.

7. Verwendung nach einem der Ansprüche 1 bis 4, **dadurch gekennzeichnet, dass** eine kationische oder anionische Verbindung hinzugemischt wird.

8. Verfahren zur Herstellung von Mischungen der in einem der Ansprüche 1 bis 7 definierten erfindungsgemäss einsetzbaren organischen UV-Filter, **dadurch gekennzeichnet, dass** man die in mikronisierter Form vorliegenden UV-Filter innig miteinander vermischt.

9. Verfahren zur Herstellung von Mischungen der in einem der Ansprüche 1 bis 6 definierten erfindungsgemäss einsetzbaren organischen UV-Filter, **dadurch gekennzeichnet, dass** die organischen UV-Filter als Gemische von mindestens zwei Einzelsubstanzen mikronisiert werden.

10. Verfahren zur Herstellung von Mischungen der in einem der Ansprüche 1 bis 7 definierten erfindungsgemäss einsetzbaren organischen UV-Filter, **dadurch gekennzeichnet, dass** mindestens zwei Einzelsubstanzen zusammengeschmolzen werden, die Schmelze abgekühlt, und der entstandene Composite anschliessend einem Mikronisierungsprozess unterworfen wird.

11. Kosmetische Formulierung, enthaltend eine Mischung aus mikronisierten organischen UV-Filtern ausgewählt aus
- Mischungen aus Octyltriazon und Methylene Bis benzotriazolyltethylbutylphenol;
- Mischungen aus 2-[(2,4-methoxy)-phenyl]-4,6-bis-[(2-hydroxy-4-methoxy)-phenyl]-(1,3,5)-triazin und Methylen-bis-benzotriazolyltetramethylbutylphenol;
- Mischungen aus Methylen-bis-benzotriazolyltetramethylbutylphenol und Dioctybutamidotriazon;
- Mischungen aus 2,2'-Methylen-bis-[6-(2H-benztriazol-2-yl)-4-methyl-phenol, Octyltriazon und der Verbindung der Formel (36) 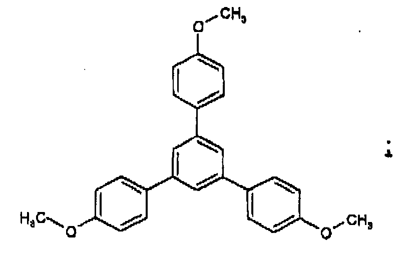
- Mischungen aus 2,2'-Methylen-bis-[6-(2H-benztriazol-2-yl)-4-methyl-phenol, Octyltriazon und der Verbindung der Formel (37) 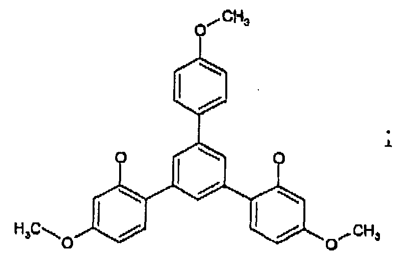
- Mischungen aus Methylen-bis-benzotriazolyltetramethylbulylphenol und der Verbindung der Formel (39 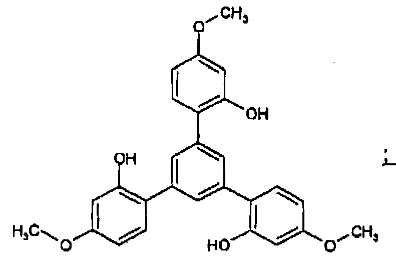 und
- Mischungen aus Methylen-bis-benzotriazolyltertamethylbuthylenol, Dioctylbutamidotriazon und der Verbindung der Formel (37) 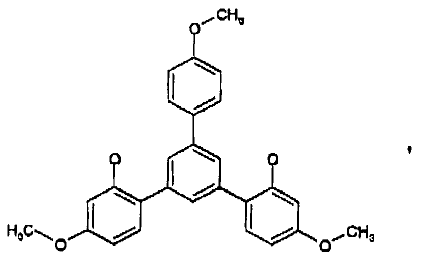
wobei die mikronisierten organischen UV-Filter eine Partikelgrösse von 0,02 bis 2 µm) aufweisen, gegebenenfalls einen oder mehrere Antioxidantien und/oder anorganische Pigmente und/oder eine kationische bzw. anionische Verbindung, sowie kosmetisch verträgliche Träger- oder Hilfsstoffe.

12. Kosmetische Formulierung nach Anspruch 11, **dadurch gekennzeichnet, dass** sie zusätzlich einen öllöslichen, nicht mikronisierten UV-Filter enthält.

## Claims

1. Use of a mixture of micronized organic UV filters selected from
- mixtures of octyl triazone and methylene bis-benzotriazolyl tetramethylbutylphenol;
- mixtures of 2-[(2,4-methoxy)-phenyl]-4,6-bis[(2-hydroxy-4-methoxy)-phenyl]-(1,3,5)-triazine and methylene bis-benzotriazolyl tetramethylbutylphenol;
- mixtures of methylene bis-benzotriazolyl tetramethylbutylphenol and dioctyl butamidotriazone;
- mixtures of 2,2'-methylene bis[6-(2H-benzotriazol-2-yl)-4-methyl-phenol, octyl triazone and the compound of formula (36) 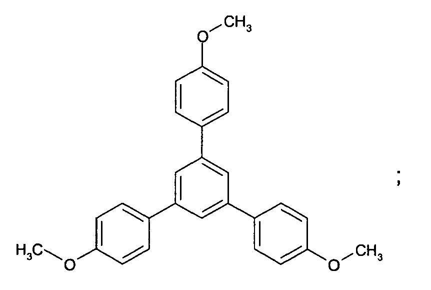
- mixtures of 2,2'-methylene bis[6-(2H-benzotriazol-2-yl)-4-methyl-phenol, octyl triazone and the compound of formula (37) 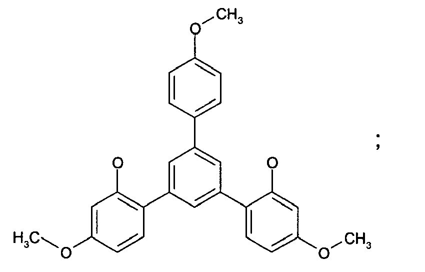
- mixtures of methylene bis-benzotriazolyl tetramethylbutylphenol and the compound of formula (39) 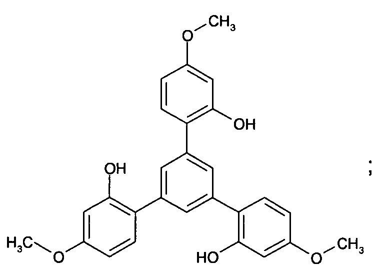 and
- mixtures of methylene bis-benzotriazolyl tetramethylbutylphenol, dioctyl butamidotriazone and the compound of formula (37) 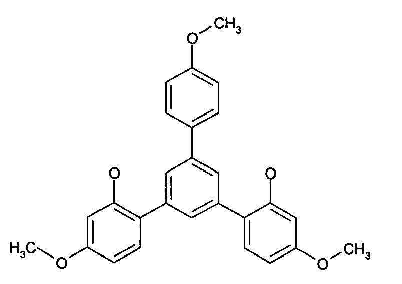
for the preparation of a pharmaceutical for protecting human and animal skin and hair against the damaging effect of UV radiation, wherein the micronized organic UV filters have a particle size from 0.02 to 2µm.

2. Use according to claim 1, wherein
- mixtures of methylene bis-benzotriazolyl tetramethylbutylphenol and octyl triazone;
- mixtures of 2-[(2,4-methoxy)-phenyl]-4,6-bis[(2-hydroxy-4-methoxy)-phenyl]-(1,3,5)-triazine and methylene bis-benzotriazolyl tetramethylbutylphenol;
- mixtures of methylene bis-benzotriazolyl tetramethylbutylphenol and dioctyl butamidotriazone; and
- mixtures of methylene bis-benzotriazolyl tetramethylbutylphenol and the compound of formula (39) 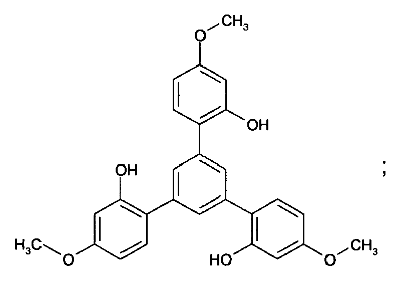
are used.

3. Use according to claim 1 or 2, wherein mixtures of methylene bis-benzotriazolyl tetramethylbutylphenol and octyl triazone; are used.

4. Use according to claim 1 or 2, wherein mixtures of methylene bis-benzotriazolyl tetramethylbutylphenol and dioctyl butamido triazone; are used.

5. Use according to any one of claims 1 to 4, wherein an inorganic pigment is used additionally.

6. Use according to any one of claims 1 to 4, wherein an antioxidant is additionally incorporated into the mixture.

7. Use according to any one of claims 1 to 4, wherein a cationic or anionic compound is incorporated into the mixture.

8. Method for the preparation of mixtures of UV filters as defined in one of the claims 1 to 7 which comprises mixing intimately the micronized organic UV absorbers.

9. Method for the preparation of mixtures of UV filters as defined in one of the claims 1 to 6 which comprises micronizing the organic UV filters as mixtures of at least two single substances.

10. Method for the preparation of mixtures of UV filters as defined in one of the claims 1 to 7 which comprises melting down at least two singles substances, cooling the cast and subjecting the obtained composite in a micronization process.

11. A cosmetic formulation, comprising a mixture of micronized organic UV filters selected from
- mixtures of octyl triazone and methylene bis-benzotriazolyl tetramethylbutylphenol;
- mixtures of 2-[(2,4-methoxy)-phenyl]-4,6-bis[(2-hydroxy-4-methoxy)-phenyl]-(1,3,5)-triazine and methylene bis-benzotriazolyl tetramethylbutylphenol;
- mixtures of methylene bis-benzotriazolyl tetramethylbutylphenol and dioctyl butamidotriazone;
- mixtures of 2,2'-methylene bis[6-(2H-benzotriazol-2-yl)-4-methyl-phenol, octyl triazone and the compound of formula (36) 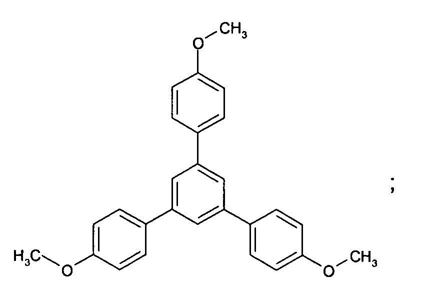
- mixtures of 2,2'-methylene bis[6-(2H-benzotriazol-2-yl)-4-methyl-phenol, octyl triazone and the compound of formula (37) 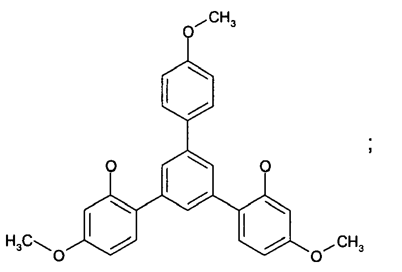
- mixtures of methylene bis-benzotriazolyl tetramethylbutylphenol and the compound of formula (39) 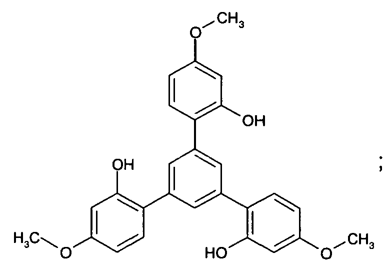 and
- mixtures of methylene bis-benzotriazolyl tetramethylbutylphenol, dioctyl butamidotriazone and the compound of formula (37) 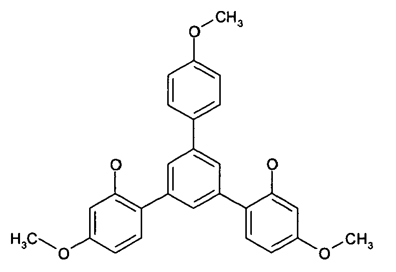
wherein the micronized organic UV filters have a particle size from 0.02 to 2µm, optionally one or more than one antioxidant and/or inorganic pigments and/or a cationic or anionic compound and cosmetically acceptable carriers or adjuvants.

12. A cosmetic formulation according to claim 11, which additionally comprises an oil-soluble, non-micronized UV filter.

## Revendications

1. Utilisation de mélanges de filtres UV organiques micronisés, choisis parmi
- des mélanges d'octyltriazone et de méthylène-bis-benzotriazolyltétraméthylbutylphénol ;
- des mélanges de 2-[(2,4-méthoxy)-phényl]-4,6-bis-[(2-hydroxy-4-méthoxy)-phényl]-(1,3,5)-triazine et de méthylène-bis-benzotriazolyltétraméthylbutylphénol ;
- des mélanges de méthylène-bis-benzotriazolyltétraméthylbutylphénol et de dioctylbutamidotriazone ;
- des mélanges de 2,2'-méthylène-bis-[6-(2H-benzotriazol-2-yl)-4-méthyl-phénol, d'octyltriazone et du composé de formule (36) 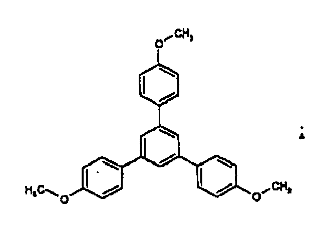
- des mélanges de 2,2'-méthylène-bis-[6-(2H-benzotriazol-2-yl)-4-méthyl-phénol, d'octyltriazole et du composé de formule (37) 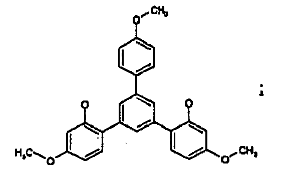
- des mélanges de méthylène-bis-benzotriazolyltétraméthylbutylphénol et du composé de formule (39) 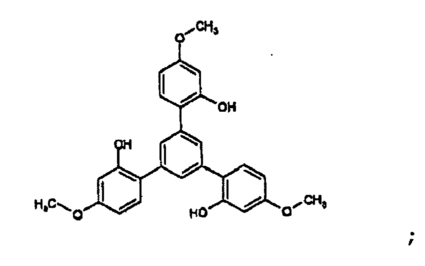 et
- des mélanges de méthylène-bis-benzotriazolyltétraméthylbutylphénol, de dioctylbutamidotriazone et du composé de formule (37) 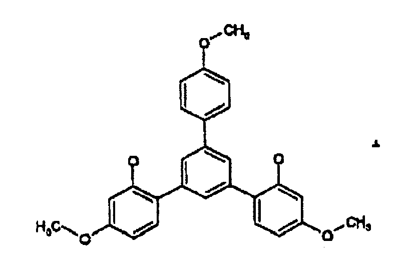
pour la fabrication d'un médicament destiné à la protection de la peau humaine et animale et des cheveux contre l'effet nocif du rayonnement UV, les filtres UV organiques micronisés ayant une taille de particule de 0,02 à 2 µm.

2. Utilisation selon la revendication 1, **caractérisé en ce qu'**on utilise
- des mélanges de méthylène-bis-benzotriazolyltétraméthylbutylphénol et d'octyltriazone ;
- des mélanges de 2-[(2,4-méthoxy)-phényl]-4,6-bis-[(2-hydroxy-4-méthoxy)-phényl]-(1,3,5)-triazine et de méthylène-bis-benzotriazolyltétraméthylbutylphénol ;
- des mélanges de méthylène-bis-benzotriazolyltétraméthylbutylphénol et de dioctylbutamidotriazone ; et
- des mélanges de méthylène-bis-benzotriazolyltétraméthylbutylphénol et du composé de formule (39)

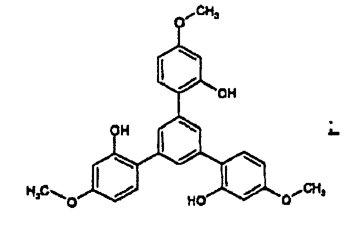

3. Utilisation selon la revendication 1 ou 2, **caractérisé en ce qu'**on utilise des mélanges de méthylène-bis-benzotriazolyltétraméthylbutylphénol et d'octyltriazone.

4. Utilisation selon la revendication 1 ou 2, **caractérisé en ce qu'**on utilise des mélanges de méthylène-bis-benzotriazolyltétraméthylbutylphénol et de dioctylbutamidotriazone.

5. Utilisation selon l'une quelconque des revendications 1 à 4, **caractérisé en ce qu'**en outre on ajoute un pigment inorganique.

6. Utilisation selon l'une quelconque des revendications 1 à 4, **caractérisé en ce qu'**en outre on ajoute un antioxydant.

7. Utilisation selon l'une quelconque des revendications 1 à 4, **caractérisé en ce qu'**on ajoute un composé anionique ou cationique.

8. Procédé pour la préparation de mélanges des filtres UV organiques utilisables selon l'invention, définis dans l'une quelconque des revendications 1 à 7, **caractérisé en ce qu'**on mélange intimement entre eux les filtres UV présents sous forme micronisée.

9. Procédé pour la préparation de mélanges des filtres UV organiques utilisables selon l'invention, définis dans l'une quelconque des revendications 1 à 6, **caractérisé en ce qu'**on micronise les filtres UV organiques sous forme de mélanges d'au moins deux substances individuelles.

10. Procédé pour la préparation de mélanges des filtres UV organiques utilisables selon l'invention, définis dans l'une quelconque des revendications 1 à 7, **caractérisé en ce qu'**on fait fondre ensemble au moins deux substances individuelles, on refroidit la masse fondue et on soumet ensuite le composite résultant à un processus de micronisation.

11. Composition cosmétique, contenant un mélange de filtres UV organiques micronisés, choisi parmi
- des mélanges d'octyltriazone et de méthylène-bis-benzotriazolyltétraméthylbutylphénol ;
- des mélanges de 2-[(2,4-méthoxy)-phényl]-4,6-bis-[(2-hydroxy-4-méthoxy)-phényl]-(1,3,5)-triazine et de méthylène-bis-benzotriazolyltétraméthylbutylphénol ;
- des mélanges de méthylène-bis-benzotriazolyltétraméthylbutylphénol et de dioctylbutamidotriazone ;
- des mélanges de 2,2'-méthylène-bis-[6-(2H-benzotriazol-2-yl)-4-méthyl-phénol, d'octyltriazone et du composé de formule (36) 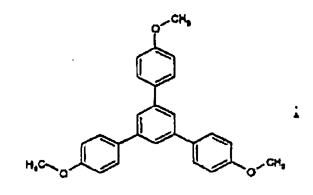
- des mélanges de 2,2'-méthylène-bis-[6-(2H-benzotriazol-2-yl)-4-méthyl-phénol, d'octyltriazole et du composé de formule (37) 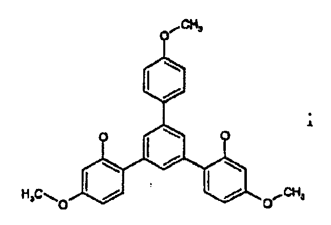
- des mélanges de méthylène-bis-benzotriazolyltétraméthylbutylphénol et du composé de formule (39) 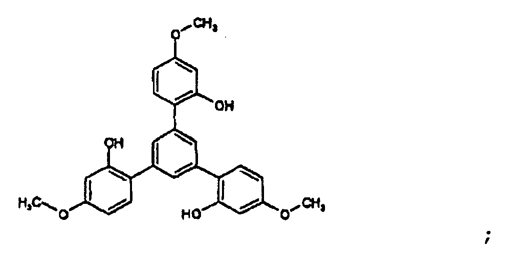 et
- des mélanges de méthylène-bis-benzotriazolyltétraméthylbutylphénol, de dioctylbutamidotriazone et du composé de formule (37) 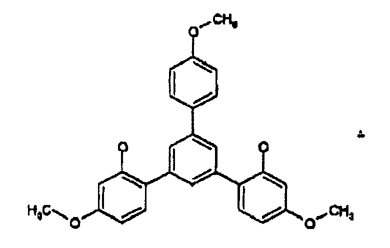
les filtres UV organiques micronisés ayant une taille de particule de 0,02 à 2 µm, éventuellement un ou plusieurs antioxydants et/ou pigments inorganiques et/ou un composé anionique ou cationique, ainsi que des véhicules ou adjuvants cosmétiquement acceptables.

12. Composition cosmétique selon la revendication 11, **caractérisée en ce qu'**en outre elle contient un filtre UV liposoluble non micronisé.
